Europäisches Patentamt

European Patent Office

Office européen des brevets

(19)

(11) Veröffentlichungsnummer: **0 186 752 B1**

# EUROPÄISCHE PATENTSCHRIFT

(45) Veröffentlichungstag der Patentschrift: **17.07.91**

(21) Anmeldenummer: **85113958.4**

(22) Anmeldetag: **02.11.85**

(51) Int. Cl.⁵: **A61B 5/103**, G01B 3/22, G01B 5/06

(54) **Vorrichtung zur Messung von Hauterhebungen.**

(30) Priorität: **14.11.84 DE 3441608**

(43) Veröffentlichungstag der Anmeldung:
**09.07.86 Patentblatt 86/28**

(45) Bekanntmachung des Hinweises auf die
Patenterteilung:
**17.07.91 Patentblatt 91/29**

(84) Benannte Vertragsstaaten:
**CH FR GB LI**

(56) Entgegenhaltungen:
**BE-A- 833 112          DE-A- 3 125 737**
**DE-A- 3 304 503          DE-B- 2 550 332**
**GB-A- 639 508            GB-A- 2 068 567**
**US-A- 4 321 752**

(73) Patentinhaber: **GESELLSCHAFT FÜR STRAHLEN- UND UMWELTFORSCHUNG M.B.H.**
**Ingolstädter Landstrasse 1 Post Oberschleissheim**
**W-8042 Neuherberg(DE)**

(72) Erfinder: **Haina, Diether, Dr.**
**Im Schäfersgarten 4**
**W-6146 Hahnlein-Alsbach(DE)**
Erfinder: **Waidelich, Wilhelm, Prof. Dr.**
**Becker-Gundulastrasse 32**
**W-8000 München 70(DE)**
Erfinder: **Landthaler, Michael, Dr.**
**Kugelfeldstrasse 23**
**W-8019 Glonn(DE)**

(74) Vertreter: **Gottlob, Peter**
**Kernforschungszentrum Karlsruhe GmbH**
**Stabs. Patente und Lizenzen Weberstrasse 5**
**W-7500 Karlsruhe 1(DE)**

## Beschreibung

Die Erfindung betrifft ein Exophytometer zur Ausmessung von nach außen herauswachsenden Läsionen und ähnlichen Erhebungen der Haut.

Aus GB-A-20 68567 ist eine Vorrichtung aus Ausmessung von Hauterhebungen bekannt, wobei eine Feder ein Abtastorgan leicht auf die Hauterhebung drückt. Die Kenntnis der Höhe von nach außen herauswachsenden Hautläsionen kann bedeutungsvoll sein zur Beurteilung der Heilungschancen und der optimalen Therapie. Das trifft beispielsweise auf das Maligne Melanom zu, eines der bösartigsten Geschwülste der Haut oder Schleimhaut. Seine Gefährlichkeit liegt in seiner starken und meist frühzeitigen Neigung zu lymphogener und/oder hämatogener Metastasenbildung mit letalem Ausgang. In den letzten Jahren wurde eine Zunahme der Häufigkeit maligner Melanome beobachtet.

Neuere Untersuchungen haben ergeben, daß ein Zusammenhang besteht zwischen der histologisch gemessenen Tumordicke und der Überlebensrate der Patienten (Brau-Falco, O., Hölzel, D.,Konz, B., Landthaler M., Schmoeckel, Ch.: Bilan therapeutique du "groupe melanome" de munich Kongreßband, XVII. Congres de l'association des dermatologistes et syphiligraphes de langue francaise, Brüssel 2.-4.6.1983, pp. 301-312). Sie liegt nach einem Zeitraum von 5 Jahren für D < 0,75 mm bei 96 %, für 0,75 mm < D < 1,5 mm bei 86 % und für 1,5 mm < D < 3 mm bei 74 %. Nur 56 % der Patienten mit malignen Melanomen, die dicker als 3 mm sind, überleben einen Zeitraum von 5 Jahren.

Die Bestimmung dieser Tumordicke ist aber erst postoperativ möglich. Nach neueren Untersuchungen erlauben aber auch klinische Kriterien praeoperativ eine Abschätzung des Metastasierungsrisikos. Wichtigstes Kriterium ist die Erhabenheit (Höhe H) des Tumors über das Hautniveau (Funk, W., Schmoeckel, Ch., Hölzel, D., Braun-Falco,O.: Low- and high-risk malignant melanome. Prognostic classification by clinical criteria. Brit.J. Dermatol. 1984 ). So ist ab einer Erhabenheit von 3 mm ein hohes Metastasierungsrisiko gegeben, d.h. bei 80 % der Patienten ist mit einer Progression zu rechnen.

Da in Zukunft die einzuschlagende Therapie vom praeoperativ festgestellten Metastasierungsrisiko beeinflußt werden wird, sollte zur optimalen Versorgung von Melanompatienten eine exakte Höhenbestimmung vorgenommen werden können.

Der Erfindung liegt nunmehr die Aufgabe zugrunde,ein Exophytometer der e.g. Art derart auszubilden, daß mit seiner Hilfe die Höhe exophytischer Hautläsionen auf einfache und schnelle Weise bestimmt werden kann, wobei der Aufwand und damit die Kosten möglichst gering gehalten werden sollen.

Diese Aufgabe wird erfindungsgemäß mittels den kennzeichnenden Merkmalen des Anspruches 1 gelöst.

Ein besonderer Vorteil der Erfindung besteht darin, daß zur Meßwertnahme z.B. ein Taststift manuell bis in Sichtkontakt auf die Hauterhebung gebracht werden kann und daß die Meßwertnahme bei Berührung des manuell oder motorisch bewegten Taststiftes mit der Hauterhebung auf elektrischem Wege signalisiert und/oder ausgelöst wird. Die Meßwertnahme kann auch berührungslos auf optischem, elektrischem oder akustischem Wege erfolgen.

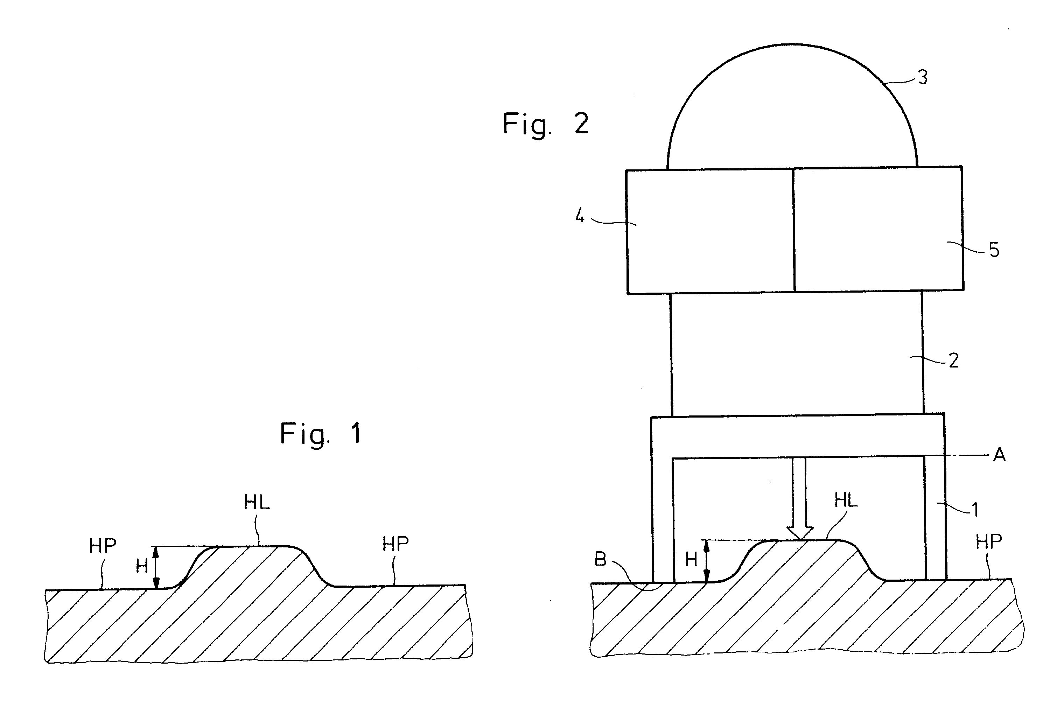

Die Erfindung wird im folgenden anhand dreier Ausführungsbeispiele mittels der Figuren 1 bis 6 näher erläutert.

Die Fig. 1 zeigt schematisch einen Schnitt durch eine exophytische Hautläsion HL, die sich über die umgebenden Hautpartien HP um die Höhe H erhebt. Diese Höhe H ist zu bestimmen, was dadurch erfolgt, daß wie in Fig. 2 schematisch dargestellt, mit Hilfe eines Abstandshalters 1 eine Vergleichsfläche A bzw. eine Bezugsfläche B in einem vorgegebenen Abstand zur Hautoberfläche HP definiert wird, von der aus die Höhe H der Hauterhebung HL mit einem Meßwertnehmer 2 als Differenz zum Abstand der Hautoberfläche HP gemessen und in der Meßwert-Anzeige 3 zur Anzeige gebracht wird. Hierbei kann entweder der Meßwert bei einem Signal des Signalgebers 4 sofort abgelesen und/oder der Meßwert in einem Meßwertspeicher 5 gespeichert und auf diese Weise zu einem späteren Zeitpunkt, beispielsweise nach Entfernen der erfindungsgemäßen Meßvorrichtung von der Haut, abgelesen werden.

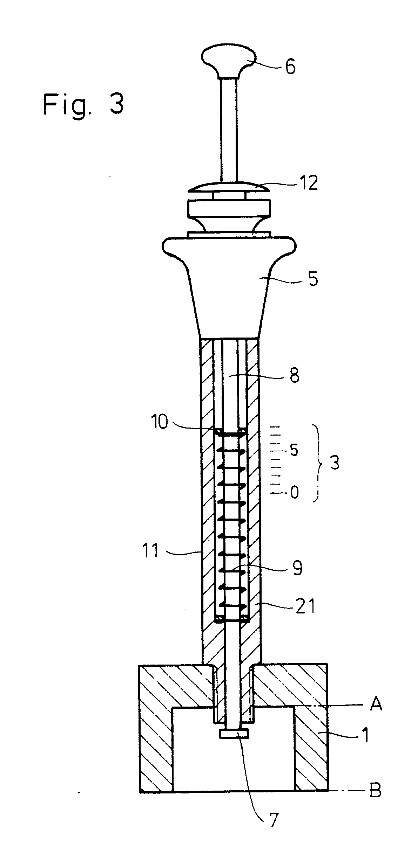

Ein Ausführungsbeispiel ist in Fig. 3 im Schnitt dargestellt.

Der Abstandshalter 1 besteht, wie auch in den übrigen Ausführungsbeispielen, aus einem Topf aus durchsichtigem Material, an dessen Boden der Meßwert-Nehmer in Form eines zylindrisch ausgebildeten Gehäuses 21 mit darin geführtem beweglichen Abtastorgan 7 angeordnet ist. Das Taststift 7 reicht durch den Boden hindurch in den Innenraum des Topfes hinein und kann relativ zur Vergleichsebene A bzw. Bezugsebene B bewegt werden. Die Bezugsebene B wird durch die Stirnkante (-fläche) des Topfes definiert. Die kann derart ausgebildet sein, daß nur Teile die Hautpartien berühren (Dreibein etc.).

Bei der in Fig. 3 gezeigten rein mechanischen, aber sehr einfachen Ausführungsform wird der Meßwert dadurch gewonnen, daß nach dem Aufsetzen des Abstandshalters 1 auf die Hautoberfläche HP ein Druckstift 6 z.B. mit einem Finger gegenüber dem Gehäuse 21 soweit heruntergedrückt wird, bis der Taststift 7 die Hauterhebung HL berührt. Druckstift 6 und Taststift 7 sind durch die Schubstange 8 starr miteinander verbunden, die durch Druck der Feder 9 auf die Nocke 10 im Gehäuse 21 nach oben gedrückt wird. Auf der Außenfläche 11 des Gehäuses 21 ist die Meßwertanzeige 3 auf die Weise eingraviert, daß bei auf die normale Hautoberfläche HP herabgefahrenem Taststift 7 der obere Rand der Nocke 10 auf den "Nullstrich" der mm-Skala 3 zeigt. Da mit dem Auge nicht gleichzeitig der Berührungspunkt von Taststift 7 und Hauterhebung HL feszustellen sowie der Meßwert ablesbar ist, wird der Meßwert dadurch gespeichert, daß die Schubstange 8 nach Loslassen des Druckstiftes 6 mechanisch festgeklemmt wird. In diesem Ausführungsbeispiel wurde dafür als Meßwert-Speicher 5 das Oberteil eines kommerziell gefertigten feststellbaren Drahtauslösers für Photoapparate verwendet. Die mechanische Sperre wird durch Betätigen des Druckringes 12 wieder gelöst.

Der Meßvorgang spielt sich folgendermaßen ab:

Das erfindungsgemäße Exophytometer wird mit dem Abstandshalter 1 auf die Hautoberfläche HP um die Hauterhebung HL aufgesetzt und dann der Druckstift 6 mit einem Finger soweit heruntergedrückt, bis der Taststift 7 die Hauterhebung HL, bzw. deren höchste Stelle, berührt . Dann wird der Druckstift 6 losgelassen und das Exophytometer von der Haut entfernt. Jetzt wird die Höhe H der Hauterhebung HL an der Stellung der Nocke 10 zur Skala an der Meßwert-Anzeige 3 abgelesen. Nach Auslösen des Druckringes 12 ist das Exophytometer für eine neue Messung bereit.

Bei Verwendung von 1/2 mm-Skalenstrichen an der Meßwert-Anzeige 3 ist die Ablesegenauigkeit durch Schätzung besser als 1/2 mm.

Je nach Beschaffenheit der Haut kann sich der Abstandshalter 1 etwas in die Hautoberfläche HP eindrücken. In diesem Falle, oder bei Messung an gekrümmten Hautoberflächen, wird zur Korrektur des Meßwertes neben der Hauterhebung HL eine Vergleichsmessung durchgeführt.

Zur Dickenmessung von Hauterhebungen verschiedenen Durchmessers können in Form und Durchmesser unterschiedliche Abstandshalter 1 verwendet werden.

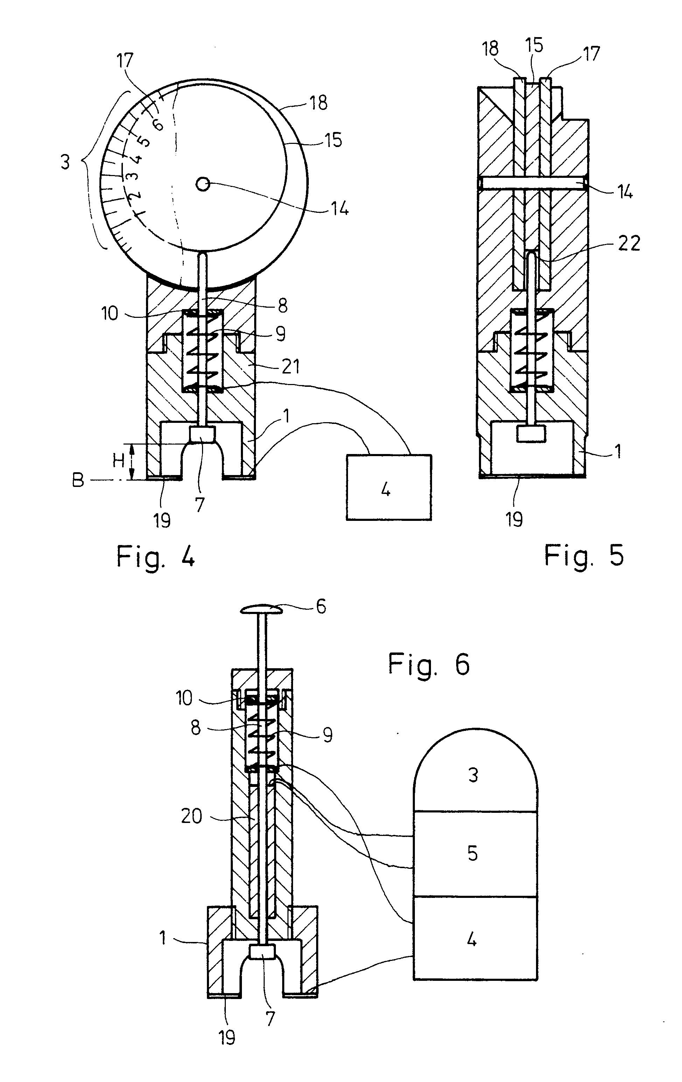

Im zweiten Ausführungsbeispiel nach Fig. 4 und 5 wird eine etwas aufwendigere Ausführungsform vorgeschlagen. Sie unterscheidet sich von der ersten dadurch, daß bei Berührung der Hauterhebung HL durch den Taststift 7 (Bezugszeichen stimmen für die entsprechenden Teile in allen Ausführungsbeispielen überein) ein Signal gegeben wird, und daß die Ablesegenauigkeit durch Verwendung eines Exzenters vergrößert ist.

Der Meßwert-Nehmer besteht wiederum aus einem Taststift 7, der gegen den Druck der Feder 9 im Gehäuse 21 über die Schubstange 8 von der Lauffläche 22 einer Exzenterscheibe 15 nach unten gegen die zu messende Hauterhebung HL gedrückt wird. Ausgelöst durch Berührung einerseits der Hautoberfläche HP mit den auf der Bezugsebene B befestigten Kontaktplatten 19 und andererseits der Hauterhebung HL mit dem Taststift 7, wird durch eine mit diesen verbundene elektronische Schaltung im Signalgeber 4 ein akustisches Signal erzeugt. Jetzt wird der Meßwert an der Meßwert-Anzeige 3 abgelesen, die von der im ersten Ausführungsbeispiel abweicht. Sie ist auf der Meßwertscheibe 17 eingraviert, die ebenso wie die Mitnehmerscheibe 18 (s.a. Fig. 5) mit der Exzenterscheibe 15 verbunden und auf der Achse 14 drehbar gelagert ist. Die Winkel, unter denen die Skalenstriche für die Höhenablesung der Meßwertanzeige 3 eingraviert sind, wurden rechnerisch ermittelt. Für kleine Höhen H ist die Meßwertanzeige genauer (bis 1 mm genauer als 0,05 mm, für Höhen über 3 mm genauer als 0,25 mm) und entspricht damit den praktischen Anforderungen.

Der Meßvorgang spielt sich folgendermaßen ab:

Das Exophytometer wird in Nullstellung mit dem mit Kontaktplatten 19 versehenen Abstandshalter 1 auf die Hautoberfläche HP um die Hauterhebung HL aufgesetzt und dann die Meßwertscheibe 17 (verbunden mit Mitnehmer- 18 und Exzenterscheibe 15) soweit gedreht, bis ein akustisches Signal im Signalgeber 4 ertönt. Jetzt wird die Höhe H an der Meßwertanzeige 3 abgelesen.

Für unregelmäßige Hautoberflächen ist sinngemäß wie unter Ausführungsbeispiel 1 zu verfahren.

Das dritte Ausführungsbeispiel gemäß Fig. 6 unterscheidet sich von den beiden anderen dadurch, daß die Höhe H der Hauterhebung HL unter Verwendung eines ohmschen Wegaufnehmers 20 ermittelt und dann elektronisch angezeigt wird. Der Taststift 7 ist (wie bei Ausführungsbeispiel 1) an der Schubstange 8 des ohmschen Wegaufnehmers 20 befestigt, die in Ruhestellung gegen den Druck der Feder 9 nach oben gedrückt wird. Durch manuelles Herunterdrücken des Druckstiftes 6 wird ein zum Weg der Schubstange 8 proportionaler elektrischer Widerstandswert erzeugt, der in der Meßwertanzeige 3 in eine Anzeige der Höhe H einer Hauterhebung HL umgewandelt wird. Sobald der Taststift 7 in Kontakt mit der Hauterhebung HL kommt (und die Kontaktplatten 19 auf der Hautoberfläche HP anliegen), wird vom Signalgeber 4 neben einem akustischen ein elektrisches Signal erzeugt, das bewirkt, daß der augenblickliche Meßwert im Meßwert-Speicher 5 gespeichert und in der Meßwert-Anzeige 3 solange angezeigt wird, bis diese für den Beginn einer neuen Messung gelöscht ist.

Die Meßgenauigkeit ist über den ganzen Meßbereich von 10 mm konstant und beträgt etwa 0,05 mm.

Der Meßvorgang spielt sich folgendermaßen ab:

Das Exophytometer wird auf die Hautoberfläche HP um die Hauterhebung HL aufgesetzt und dann der Druckstift 6 heruntergedrückt bis ein Signal ertönt. Der Meßwert kann jetzt digital abgelesen werden.

Der Vorteil dieser Ausführungsform liegt darin, daß der Druckstift 6 zur Ablesung des Meßwertes nicht festgehalten werden muß, weil dieser im Moment der Berührung der Hauterhebung HL durch den Taststift 7 ermittelt und gespeichert wird.

## Patentansprüche

1. Exophytometer zur Ausmessung von nach außen herauswachsenden Läsionen und ähnlichen Erhebungen der Haut, mit
   a) einem Abtastorgan (7), welches relativ und senkrecht zu einer Bezugsebene (B) bewegbar ist,
   b) einer mit dem Abtastorgan (7) starr verbundene Schubstange (8) in einem zylindrisch ausgebildeten Meßwert-Nehmergehäuse (21) und
   c) einem Abstandshalter (1), der die Position des Abtastorgans relativ zur Bezugsebene B definiert und mit dem Gehäuse (21) verbunden ist
   gekennzeichnet, durch
   d) eine Feder (9), welche die Schubstange (8) im Meßwert-Nehmergehäuse (21) derart nach oben drückt, daß nur Ausmessung des Erhebung das Abtastorgan gegen die Kraft der Feder bewegt werden muß

2. Exophytometer nach Anspruch 1, gekennzeichnet durch den Abstandshalter (1), der derart ausgebildet ist, daß zumindest drei Bereiche seiner, die Bezugsebene (B) bildenden Stirnfläche an unterschiedlichen Stellen der Hautpartien (HP) um die Läsionen oder Erhebungen (HL) herum zur Auflage kommen und daß das Abtastorgan (7) am Abstandshalter (1) in definierter Stellung zur Bezugsebene (B) befestigt ist.

3. Exophytometer nach Anspruch 1 und 2, dadurch gekennzeichnet, daß der Abstandshalter (1) ein Topf ist, der über die Läsionen und/oder Erhebungen (HL) gestülpt ist, daß der Rand des Topfes die Bezugsebene (B) bildet und daß das Abtastorgan (7) ein Stift ist, der im Zentrum des Bodens des Topfes in den Innenraum des Topfes hineinbewegbar ist.

4. Exophytometer nach Anspruch 1 oder einem der folgenden, dadurch gekennzeichnet, daß für den Meßwert-Nehmer (1, 7) mechanische, elektrische, optische oder akustische Längenmeßverfahren verwendbar sind.

5. Exophytometer nach Anspruch 1 oder einem der folgenden, dadurch gekennzeichnet, daß die Berührung des Taststiftes (7) mit der Hauterhebung (HL) auf optischem oder elektrischem Wege feststellbar ist.

6. Exophytometer nach Anspruch 1 oder einem der folgenden, dadurch gekennzeichnet, daß ein Signalgeber (4) bei Berührung des Taststiftes (7) mit der Hauterhebung (HL) optische, akustische und/oder elektronische Signale abgibt.

7. Exophytometer nach Anspruch 1 oder einem der folgenden, dadurch gekennzeichnet, daß die gemessenen Werte (H) auf mechanischem oder elektrischem Wege speicherbar sind.

8. Exophytometer nach Anspruch 1 oder einem der folgenden, dadurch gekennzeichnet, daß die Speicherung der Meßwerte (H) vom Signalgeber (4) steuerbar ist.

## Claims

1. Exophytometer for measuring outwardly growing lesions and similar protuberances of the skin, having
   a) a scanning means (7), which is displaceable relative and perpendicular to a reference plane (B),
   b) a push rod (8), which is rigidly connected to the scanning means (7) and located in a cylindrically shaped transducer housing (21), and
   c) a spacer member (1), which defines the position of the scanning means relative to the reference plane (B) and is connected to the housing (21),
   characterised by
   d) a spring (9), which presses the push rod (8) in the transducer housing (21) upwardly in such a manner that, to measure the protuberance, the scanning means must be moved in a direction in opposition to the force of the spring.

2. Exophytometer according to claim 1, characterised by the spacer member (1), which is so adapted that at least three regions of its end face forming the reference plane (B) are supported at different locations on the skin portions (HP) around the lesions or protuberances (HL), and the scanning means (7) is mounted on the spacer member (1) in a defined position relative to the reference plane (B).

3. Exophytometer according to claims 1 and 2, characterised in that the spacer member (1) is a container, which is inverted above the lesions and/or protuberances (HL), the edge of the container forms the reference plane (B), and the scanning means (7) is a pin, which is insertable into the interior of the container in the centre of the base of the container.

4. Exophytometer according to claim 1 or one of the preceding claims, characterised in that mechanical, electrical, optical or acoustical length measuring processes are utilisable for the transducer (1, 7).

5. Exophytometer according to claim 1 or one of the preceding claims, characterised in that the contact of the feeler pin (7) with the skin protuberance (HL) is detectable in an optical or electrical manner.

6. Exophytometer according to claim 1 or one of the preceding claims, characterised in that a signal transmitter (4) transmits optical, acoustical and/or electronic signals upon the feeler pin (7) contacting the skin protuberance (HL).

7. Exophytometer according to claim 1 or one of the preceding claims, characterised in that the measured values (H) are storable in a mechanical or electrical manner.

8. Exophytometer according to claim 1 or one of the preceding claims, characterised in that the storage of the measured values (H) is controllable by the signal transmitter (4).

**Revendications**

1. Exophytomètre de mesure de lésions poussées vers l'extérieur et protubérances analogues de la peau, avec :
   a) un organe de palpage (7), mobile relativement et perpendiculairement à un plan de référence (B),
   b) une tige coulissante (8) liée solidairement à l'organe de palpage (7) dans un boîtier-capteur de mesure de forme cylindrique (21) et,
   c) un support (1) qui définit la position de l'organe de palpage par rapport au plan de référence (B) et qui est relié au boîtier (21), caractérisé par :

y a un ressort (9) qui appuie sur la tige coulissante (8) vers le haut dans le boîtier-capteur de mesure (21), de sorte qu'on doit déplacer l'organe de palpage en opposition à la force du ressort pour mesurer la protubérance.

2. Exophytomètre selon la revendication 1, caractérisé en ce que le support (1) est constitué de telle façon que trois zones au moins de sa face frontale formant le plan de référence (B) viennent en appui en des endroits différents des parties de peau (HP) autour des lésions ou protubérances (HL) et que l'organe de palpage (7) est fixé sur le support (1) dans une position déterminée par rapport au plan de référence (B).

3. Exophytomètre selon la revendication 1 et 2, caractérisé en ce que le support (1) est un pot qu'on renverse sur les lésions et/ou les protubérances (HL), le bord du pot formant le plan de référence (B) et l'organe de palpage (7) étant une tige qui peut se déplacer au centre du fond du pot dans l'espace interne du pot.

4. Exophytomètre selon la revendication 1 ou l'une des suivantes, caractérisé en ce qu'on peut utiliser pour le capteur de mesure (1, 7) des procédés mécaniques, électriques, optiques ou acoustiques.

5. Exophytomètre selon la revendication 1, ou l'une des suivantes, caractérisé en ce que par des moyens optiques ou électriques pour mettre en évidence le contact de la tige de palpage (7) avec la protubérance de peau (HL) .

6. Exophytomètre selon la revendication 1 ou l'une des suivantes, caractérisé par un émetteur de signal (4) qui émet des signaux optiques, acoustiques et/ou électriques lors du contact de la tige de palpage (7) avec la protubérance de peau (HL).

7. Exophytomètre selon la revendication 1 ou l'une des suivantes, caractérisé en ce que par des moyens mécaniques ou électriques pour enregistrer les valeurs mesurées (H) .

8. Exophytomètre selon la revendication 1 ou l'une des suivantes, caractérisé en ce que l'émetteur de signal (4) commande l'enregistrement des mesures (H).

Fig. 2

Fig. 1

6

Fig. 3

Fig. 4

Fig. 5

Fig. 6